# EUROPEAN PATENT APPLICATION

(11) **EP 3 572 076 A1**
(43) Date of publication of application: **27.11.2019**
(21) Application number: 18196621.9
(22) Date of filing: 25.09.2018
(51) Int. Cl.: A61K 9/48, A61K 31/192, A61P 29/02

(54) **PROCESS FOR PREPARING PHARMACEUTICAL COMPOSITIONS FOR SOFT GELATIN FORMULATIONS**

(30) Priority: 25.05.2018 IN 201841019620
(71) Applicant: Strides Shasun Limited, 560076 Bangalore (IN)
(72) Inventor: RAMACHANDRAPPA, Anil Kumar, 560076 Bangalore (IN); PRADEEP, Gattimallanahalli Channabasappa, 572138 Tumkur (IN)
(74) Representative: Rees, Kerry

(57) **Abstract**

The invention disclosed herein is a process for increasing the desired concentration of a pharmaceutically active ingredient relative to fill composition viscosity for dosage units. The process is particularly useful in the preparation of soft gelatin capsules containing pharmaceutically active ingredient. As a result of the process, lesser quantities of inactive ingredients are needed to accomplish the same therapeutically effective dosage, thereby significantly increasing the concentration of the pharmaceutically active ingredient resulting in either a reduction in overall fill volume and dosage unit size or an increase in concentration of pharmaceutically active ingredient per unit dosage form.

## Description

### FIELD OF THE INVENTION:

The invention disclosed herein relates to the field of oral pharmaceutical compositions. In particular, the invention relates to an improved process for preparing pharmaceutical compositions for use in soft gel formulations. The inventive process allows for a desired dosage amount of active ingredient to be placed in a smaller acceptable fill volume of dosage unit.

### BACKGROUND OF THE INVENTION:

Filled one piece soft gels have been widely known and used for many years and for a variety of purposes. Because softgels have properties which are different from conventional telescoping two-piece hard shell capsules, the soft gels are capable of retaining liquid fill material. Typically, softgels are used to contain orally consumable materials such as vitamins and pharmaceutical compositions in a liquid vehicle or carrier.

In general, not all liquids are suitable as vehicles or carriers for inclusion in softgels. For example, water, propylene glycol, glycerin, low molecular weight alcohols, ketones, acids, amines and esters cannot be used as a carrier in softgels by themselves since they interact with the gel and, if present, they can only be present in relatively small amounts.

Another limitation associated with softgels is the ability to incorporate a single dose of the pharmaceutically active ingredient in solution in an acceptable fill volume. Often, it is difficult to dissolve the pharmaceutically active ingredient in a volume of solvent small enough to produce a softgel which delivers the desired dosage amount, is economically appropriate and comfortable to ingest by the patient. Developing solvent systems for pharmaceutically active ingredients that neither significantly interact with the active ingredient nor the softgel casing itself, has proven a difficult art.

The chemical properties of certain types of drugs have necessitated the development of special solvent systems for soft gel dosage forms. Yu et al., Australian Patent Application No. 81573/87 discloses pharmaceutical formulations suitable for filling soft gels comprising acidic pharmaceutical agents and solvent systems, the solvent systems comprising 10% to 80% by weight polyethylene glycol, 1% to 20% by weight water and hydroxide ion species. The solvent systems dissolve the pharmaceutical agent, e.g., ibuprofen, in concentrations sufficient for use in soft gelatin capsules.

Increasing the concentrations of active ingredients in soft gelatin dosage forms and/or units without necessitating an increase in overall fill volume (and thereby increasing overall size of the dosage form) and/or without increased disintegration of the gelatin casing have proven difficult to accomplish in the art. Also another problematic issue is that the maintenance of a workable viscosity during such processes. Hence, there exists a need for improved processes in the pharmaceutical industry which produce pharmaceutical formulations in a manner which are more economical to manufacture and increase patient comfort.

### SUMMARY OF THE INVENTION:

The invention herein provides for a process whereby the concentration of pharmaceutically active ingredients in soft gelatin dosage units can be increased, thereby permitting the use of reduced overall fill volumes or, alternatively, higher concentrations of the active ingredient per unit dosage form. Furthermore, undesirable interactions between the fill ingredients and the gelatin casing can be reduced or altogether avoided by using the said process of the invention.

The process according to the invention increases the achievable concentration of a pharmaceutically active ingredient relative to fill viscosity for use in soft gelatin dosage units comprises the addition of pharmaceutically active ingredient and a hydroxide ion source to polyethylene glycol.

Accordingly, the invention discloses a process of increasing the concentration of acidic pharmaceutically active ingredient relative to fill composition viscosity for soft gelatin formulations comprising the steps of:
a) Dissolving complete quantity of hydroxide ion source to be used in the fill composition in purified water;
b) Adding the complete quantity of hydroxide ion source that is used in the fill composition of step a) to the total amount of PEG or their derivatives to be used in the fill composition followed by mixing to form clear solution; and
c) Adding complete quantity of the acidic pharmaceutically active ingredient to be used in the fill composition to the solution of step b) followed by mixing until complete dissolution is achieved.

Another embodiment of the invention, discloses a process of increasing the concentration of acidic pharmaceutically active ingredient relative to fill composition viscosity for soft gelatin formulations comprising the steps of:
a) Dissolving complete quantity of hydroxide ion source to be used in the fill composition in purified water;
b) Combining a first portion of pharmaceutically active ingredient with the total amount of polyethylene glycol to be used in the fill composition to form a viscous slurry;
c) Adding contents of step, a) to the viscous slurry formed in step b) and mixing until dissolved to form clear solution; and
d) Combining a second portion of pharmaceutically active ingredient to the clear solution of step c) and mixing until dissolved to form clear solution.

The resulting fill composition contains the acidic pharmaceutically active ingredient in a solvent system which is particularly suitable in the preparation of soft gelatin capsules, and permits higher doses of active ingredient to be administered without increasing overall fill volume and thereby dosage unit size. Alternatively, the resulting fill compositions permit increased concentrations of pharmaceutically active ingredient to be used per dosage unit size. Pharmaceutically active ingredients suitable for use in the invention include, but are not limited to, acidic compounds such as ibuprofen, naproxen, indomethacin, acetaminophen and diphenhydramine and combinations thereof. A preferred pharmaceutically active ingredient is ibuprofen.

The solvent system prepared in accordance with the invention comprises polyethylene glycol (PEG) and a hydroxide ion source. Polyethylene glycols which can be used in accordance with the invention include those having a molecular weight range from about 200 Daltons to about 100,000 Daltons, and preferably ranging from about 400 Daltons to about 700 Daltons. Suitable hydroxide ion sources for use in the invention include sodium hydroxide (NaOH) and potassium hydroxide (KOH), more preferably potassium hydroxide. The polyethylene glycol used herein has an average molecular weight in the range of about 200-100,000 daltons (hereinafter, all molecular weights are expressed in daltons), Moreover, the weight of polyethylene glycol selected affects the type of solution produced. Polyethylene glycol having an average molecular weight from about 200-800, preferably from about 400-700, and most preferably about 600, produces a softgel fill solution that is a liquid. Polyethylene glycol having an average molecular weight from about 800-10,000, preferably from about 2,000-8,000, produces a softgel fill solution that is semi solid, and polyethylene glycol having an average molecular weight about 10,000-100,000, preferably about 15,000-60,000, produces a softgel fill solution that is solid.

According to the process of the invention, a complete quantity of pharmaceutically active ingredient to be used in the fill composition is combined with polyethylene glycol and mixed together to form a viscous slurry. A complete quantity of hydroxide ion source to be used in the fill composition is added to the viscous slurry and mixed to form a solution.

According to the process of the invention, a first portion of the pharmaceutically active ingredient is combined with polyethylene glycol and mixed together to form a viscous slurry. A complete quantity of hydroxide ion source to be used in the fill composition is added to the viscous slurry and mixed to form a solution. The second portion of the pharmaceutically active ingredient is added to the solution and mixed until dissolved to form solution. The first portion and the second portion of the pharmaceutically active ingredient together comprises the total amount used to prepare the liquid fill composition.

The invention also provides for a soft gelatin capsule containing a fill composition prepared according to the process of the invention.

In another embodiment, the invention provides fill composition for gelatin capsules which comprises:
a) An acidic pharmaceutically active ingredient in the range of about 50% to 55% by weight, of the total fill composition;
b) Polyethylene glycol in the range of about 30 to 40% by weight of the total fill composition.; and
c) A hydroxide ion source in the range of about 5.5% or less by weight of the total fill composition.

Preparing a liquid fill composition according to the process of the invention increases the achievable concentration of pharmaceutically active ingredient in the solvent system for a given viscosity. One advantage of the invention is that lesser quantities of the ingredients for the fill composition other than the pharmaceutically active ingredient can be used. For example, smaller quantities of polyethylene glycol are needed for the same amount of active ingredient. Hence, the same dosage of pharmaceutically active ingredient can be accomplished using smaller overall fill volume as compared to previous techniques. The invention can be used to render manufacturing processes more economical and improves patient comfort by reducing capsule size or the number of dosage units needed for treatment.

### DETAILED DESCRIPTION OF THE INVENTION:

As used herein, the term "soft gelatin dosage unit" is intended to encompass any dosage unit and/or form which employs a gelatin or gelatin-like casing. Numerous casing materials have been proposed for soft capsules including gums, carrageenans, hydroxypropylated starches, celluloses, and the like. As used herein, the term "soft gelatin dosage unit" means a dosage form constructed of mammalian gelatin, fish gelatin, gums, guars, carrageenans, modified starches and the like.

The terms "fill" and "fill composition" are meant to describe that portion of a dosage unit (e.g., pill, capsule, and the like) that is encased or otherwise contained within the outermost portion. When used in reference to soft gelatin dosage units, the terms refer to compositions encased inside the gelatin containment.

The general steps of the process of the invention comprise complete addition of the pharmaceutically active ingredient to be used in the fill composition to the total polyethylene glycol to be used in the fill composition. In the first step of the process, complete quantity of pharmaceutically active ingredient to be used in the fill composition is combined with all of the polyethylene glycol, to be used in the fill composition and mixed to form a viscous slurry. Subsequently, a complete quantity of hydroxide ion source to be used in the fill composition is added to the viscous slurry and the ingredients are mixed until dissolved to form a solution.

The general steps of the process of the invention comprise adding first portion of pharmaceutically active ingredient to the total polyethylene glycol to be used in the fill composition. In the first step of the process, a first portion of pharmaceutically active ingredient is added to the total polyethylene glycol to be used in the fill composition and mixed to form viscous slurry. Subsequently, a complete quantity of hydroxide ion source to be used in the fill composition is added to the viscous slurry and the ingredients are mixed until dissolved to form a solution. To the solution added, a second portion of pharmaceutically active ingredient and mixed to form solution.

The addition of the pharmaceutically active ingredient as described produces a solvent system for the active ingredient which balances the interaction between the active ingredient and the viscosity of the fill in such a manner that accommodates higher concentrations of the active ingredient per total volume of fill without creating excessively high viscosities.

Pharmaceutically active ingredients useful in the present invention include acidic compounds such as ibuprofen, naproxen, indomethacin, and acetaminophen. A preferred pharmaceutically active ingredient is ibuprofen.

The hydroxide ion source used in the invention is generally present in an amount of about 5.5% or less of the total fill composition volume, since degradation of gelatin casings tends to occur above about 5.5% hydroxide content. Suitable hydroxide ion sources include, but are not limited to, potassium hydroxide and sodium hydroxide. A preferred hydroxide ion source is potassium hydroxide. Most preferred for use in the invention is a 50% aqueous solution of potassium hydroxide. Potassium hydroxide is preferred as the hydroxide ion source because it enhances the solubility of acidic pharmaceutical ingredients more than sodium hydroxide and is less likely to result in precipitation over a wide variety of concentrations at lower temperatures.

The initial suspension used in the process typically contains the total amount of polyethylene glycol which will be used for the fill composition. Polyethylene glycols (PEG) which can be used in accordance with the invention include those having a molecular weight range from about 200 Daltons to about 100,000 Daltons, and preferably ranging from about 400 Daltons to about 700 Daltons.

In an alternative embodiment, polyethylene glycol derivatives can be used in accordance with the invention. Suitable polyethylene glycol derivatives include, but are not limited to, polyethylene glycol ethers of alcohols and co-polymers of polyethylene glycol. An example of a polyethylene glycol ether of an alcohol is tetraglycol, which is a polyethylene glycol ether of tetrahydrofurfuryl alcohol.

In an alternative embodiment, other solvent systems can be used in accordance with the invention. For example, suitable solvent systems include those described in Makino et al. U.S. Pat. No. 5,912,011 and Morton et al. U.S. Pat. No. 5,376,688, the entire texts of which are incorporated herein by reference.

Additional ingredients which enhance the solubility of the active pharmaceutical ingredient in polyethylene glycol can be used as well, provided such ingredients are present only in amounts sufficient to preserve the desired viscosity and that do not degrade the gelatin capsule. Examples of additional ingredients include, but are not limited to, glycerin, propylene glycol, and polyvinylpyrrolidone, and combinations thereof. The amount and combination of additional ingredient(s) used will vary according to the chemical properties of the other ingredients used in the process.

### EXAMPLE 1

### Processes for Preparing Fill Compositions Containing Ibuprofen

### Process 1 was carried out according to the invention as follows:

| **Ingredients** | **Qty/batch 20,000caps** |
|---|---|
| Ibuprofen | 4.0kg |
| Potassium Hydroxide | 0.512kg |
| PEG 600 | 1.948 kg |
| Purified Water | 0.34kg |

1. Dissolved complete 0.512kg quantity of potassium hydroxide to be used in the fill composition into complete 0.34kg quantity of purified water to be used in the fill composition into a SS vessels. and cooled to room temperature about 25°C.
2. Transferred complete 1.948 kg quantity of PEG 600 to be used in the fill composition into mixing vessels and added to the contents of step 1 and mixed until a clear solution is obtained.
3. Added complete 4.0kg quantity of Ibuprofen into contents of step 2 and mixed until a clear solution is formed.

### Process 2 was carried out according to the invention as follows:

| **Ingredients** | **Qty/batch 5,000caps** |
|---|---|
| Ibuprofen | 1.0kg |
| Potassium Hydroxide | 0.128kg |
| PEG 600 | 0.487kg |
| Purified Water | 0.085kg |

1. Dissolving complete 0.128kg quantity of potassium hydroxide to be used in the fill composition into complete 0.085kg quantity of purified water to be used in the fill composition into a SS vessels and cooled to room temperature about 25°C.
2. Transferred complete 0.487kg quantity of PEG 600 to be used in the fill composition into mixing vessels and added first portion of around 0.5kg of Ibuprofen and mixed to get a viscous slurry.
3. Transferred the contents of step 1 into step 2 and mixed until a clear solution is obtained.
4. Added second portion of around 0.5kg of Ibuprofen into contents of step 3 and mixed until clear solution is obtained.

### Industrial Applicability

When the process of the invention is used in manufacturing soft gelatin dosage units, the solubilization of pharmaceutically active ingredients such as ibuprofen can be significantly increased thereby permitting smaller fill volumes for a given dosage to be employed. Accordingly, smaller capsule sizes or fewer capsules need to be produced, thereby allowing more economical, cost effective manufacturing and improving patient comfort and compliance.

The complete disclosures of all patents, patent applications, and publications are incorporated herein by reference as if each were individually incorporated by reference. The invention has been described with reference to various specific and preferred embodiments and techniques. However, it should be understood that reasonable variations and modifications are possible from the foregoing disclosure without departing from either the spirit or scope of the present invention as defined by the claims.

## Claims

1. A process of increasing the concentration of an acidic pharmaceutically active ingredient relative to fill composition viscosity for soft gelatin units comprising steps of:
a) Dissolving complete quantity of hydroxide ion source to be used in the fill composition in purified water;
b) Adding complete quantity of hydroxide ion contents of step a) to total amount of PEG or their derivatives to be used in fill composition followed by mixing to form a clear solution;
c) Adding complete quantity of the pharmaceutically active ingredient to the fill composition of step b) followed by mixing to form the solution.

2. A process of increasing the concentration of an acidic pharmaceutically active ingredient relative to fill composition viscosity for soft gelatin units comprising steps of:
a) Adding first portion of pharmaceutically active ingredient to the total amount of PEG or their derivatives to form a viscous slurry;
b) Dissolving complete quantity of hydroxide ion source to be used in the fill composition in purified water and adding complete quantity of hydroxide ion contents to step a);
c) Adding second portion of pharmaceutically active ingredient to the step b) followed by mixing until dissolved to form the solution.

3. The process as claimed in claim 1, wherein the fill composition for soft gelatin unit comprises:
a) An acidic pharmaceutically active ingredient in the range of about 50% to 55% by weight of the total fill composition;
b) Polyethylene glycol or their derivatives in the range of about 30 to 40% by weight of the total fill composition;
c) A hydroxide ion source in the range of about 5.5% or less by weight of the total fill composition; and
d) Purified water

4. The process as claimed in claim 1, wherein the acidic pharmaceutically active ingredient is selected from the group consisting of Ibuprofen, Naproxen, Indomethacin, Acetaminophen and Diphenhydramine and combinations thereof.

5. The process as claimed in claim 4, wherein the pharmaceutically active ingredient is Ibuprofen.

6. The process as claimed in any one of the preceding claims, wherein the PEG or their derivatives have an average molecular weight in the range of about 200 Daltons to 800 Daltons.

7. The process as claimed in claim 1, wherein the hydroxide ion source is selected from sodium hydroxide (NaOH) or potassium hydroxide (KOH).

8. The process as claimed in claim 7, wherein the hydroxide ion source is KOH.

9. A fill composition with high concentration of acidic pharmaceutical ingredient relative to fill composition viscosity for soft gelatin units comprises:
a) An acidic pharmaceutically active ingredient in the range of about 50 to 55 % by weight of the total fill composition;
b) Polyethylene glycol or their derivative in the range of about 30 to 40% by weight of the total fill composition;
c) A hydroxide ion source in the range of about 5.5% or less by weight of the total fill composition; and
d) Purified water.

10. The fill composition as claimed in claim 1, wherein the acidic pharmaceutically active ingredient is selected from the group consisting of Ibuprofen, Naproxen, Indomethacin, Acetaminophen, and Diphenhydramine and combinations thereof.

11. The fill composition as claimed in claim 1, wherein, the acidic pharmaceutically active ingredient is Ibuprofen.
